(19) Europäisches Patentamt
European Patent Office
Office européen des brevets

(11) Numéro de publication: **0 147 312**
**B1**

(12) # FASCICULE DE BREVET EUROPÉEN

(45) Date de publication du fascicule du brevet: **19.04.89**

(21) Numéro de dépôt: **84402661.7**

(22) Date de dépôt: **19.12.84**

(51) Int. Cl.⁴: **C 07 D 487/04,** A 61 K 31/505 // (C07D487/04, 239:00, 235:00)

(54) **Nouvelles imidazo /1,2-c/ pyrimidines et leurs sels, leur procédé de préparation, leur application à titre de médicaments et les compositions les renfermant.**

(30) Priorité: **22.12.83 GB 8334210**

(43) Date de publication de la demande: **03.07.85 Bulletin 85/27**

(45) Mention de la délivrance du brevet: **19.04.89 Bulletin 89/16**

(84) Etats contractants désignés: **AT BE CH DE FR IT LI LU NL SE**

(56) Documents cité:
**EP-A-0 061 380**
**EP-A-0 104 104**
**FR-A-2 263 776**

(73) Titulaire: **ROUSSEL- UCLAF, 35, boulevard des Invalides, F-75007 Paris (FR)**

(72) Inventeur: **Tully, Wilfred Roger, 5 Saint Peter's Road, Cirencester Gloucestershire (GB)**

(74) Mandataire: **Vieillefosse, Jean- Claude, Département des Brevets ROUSSEL UCLAF B.P no 9, F-93230 Romainville (FR)**

**EP 0 147 312 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne des imidazo[1,2-c] pyrimidines et leurs sels, ainsi que le procédé de préparation, l'application à titre de médicament de ces nouveaux produits et les compositions les renfermant.

La demande de brevet européen EP-A-0 104 104 décrit des imidazo[1,2-a] pyrimidines présentant des propriétés anxiolytiques.

L'invention a pour objet des imidazo[1,2-c] pyrimidines répondant à la formule (I):

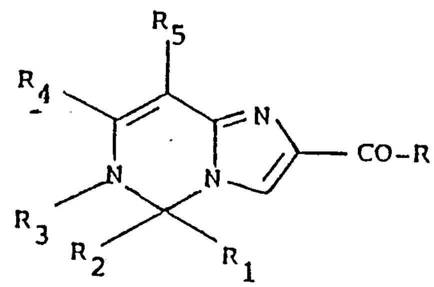

(I)

dans laquelle

R représente un radical aryle renfermant de 6 à 12 atomes de carbone,

$R^1$ représente un atome d'hydrogène, un radical alkyle, alkoxy ou alkylthio renfermant de 1 à 5 atomes de carbone ou forme avec $R_2$ une fonction cétone,

$R_2$ forme avec $R_1$ une fonction cétone ou $R_2$ forme avec $R_3$ une seconde liaison carbone-azote,

$R_3$ forme avec $R_2$ une seconde liaison carbone-azote ou $R_3$ représente un atome d'hydrogène, un radical alkyle renfermant de 1 à 5 atomes de carbone ou un radical alcényle renfermant de 2 à 5 atomes de carbone,

$R_4$ représente un radical alkoxy renfermant de 1 à 5 atomes de carbone ou un radical alkylthio renfermant de 1 à 5 atomes de carbone,

$R_5$ représente un atome d'hydrogène ou un radical alkyle, renfermant de 1 à 5 atomes de carbone,

ainsi que leurs sels d'addition avec les acides minèraux ou organiques.

Dans la formule générale (I) et dans ce qui suit:

- le terme radical alkyl, renfermant de 1 à 5 atomes de carbone désigne par exemple un radical méthyle, éthyle, propyle, isopropyle, butyle, isobutyle, tertbutyle ou pentyle;
- le terme radical alkoxy renfermant de 1 à 5 atomes de carbone désigne par exemple un radical méthoxy, éthoxy, propoxy, isopropoxy, butoxy, isobutoxy, tertbutoxy ou pentoxy;
- le terme radical alkylthio renfermant de 1 à 5 atomes de carbone désigne par exemple un radical méthylthio, éthylthio, propylthio, isopropylthio, butylthio, isobutylthio, tertbutylthio ou pentylthio;
- le terme radical aryle renfermant de 6 à 12 atomes de carbone désigne par exemple un radical phényle ou naphtyle;
- le terme radical alcényle renfermant de 2 à 5 atomes de carbone désigne par exemple un radical allyle ou vinyle.

Les sels d'addition avec les acides minéraux ou organiques peuvent être, par exemple, les sels formés avec les acides chlorhydrique, bromhydrique, iodhydrique, nitrique, sulfurique, phosphorique, propionique, formique, benzoïque, maléique, fumarique, succinique, tartrique, citrique, oxalique, glyoxylique, aspartique, alcanesulfoniques tels que l'acide méthanesulfonique et arylsulfoniques, tels que l'acide benzènesulfonique.

Parmi les produits, objet de l'invention, on peut citer notamment les dérivés répondant à la formule (I) ci-dessus, ainsi que leurs sels, caractérisés en ce que dans ladite formule (I), R représente un radical phényle, $R_1$, $R_2$, $R_3$, $R_4$ et $R_5$ ont la signification déjà indiquée.

Parmi ces derniers, on peut citer les dérivés répondant à la formule (I) ci-dessus, ainsi que leurs sels, caractérisés en ce que dans ladite formule (I), $R_4$ représente un radical méthoxy ou méthylthio et $R_1$, $R_2$, $R_3$ et $R_5$ ont la signification déjà indiquée.

Parmi les produits, objet de l'invention, on retient tout particulièrement les produits de formule (I) dans laquelle $R_3$ forme avec $R_2$ une seconde liaison carbone-azote ou représente un radical alkyle renfermant de 1 à 5 atomes de carbone ou un radical alcényle renfermant de 2 à 5 atomes de carbone, $R_4$ représente un radical méthoxy ou méthylthio et R représente un radical phényle et notamment:

- la 2-benzoyl-6-éthyl-7-méthoxy-imidazo[1,2-c] pyrimidin-5-one;
- la 2-benzoyl-7-méthoxy-6-propyl-imidazo[1,2-c] pyrimidin-5-one;
- la (5-éthyl-7-méthoxy-imidazo[1,2-c] pyrimidin-2-yl) phényl méthanone;
- la (7-méthoxy-5-méthyl imidazo[1,2-c] pyrimidin-2-yl) phényl méthanone;
- la 6-allyl-2-benzoyl-7-méthoxyimidazo[1,2-c] pyrimidin-5-one;
- la (7-méthoxy-5-méthylthio-imidazo[1,2-c] pyrimidin-2-yl) phényl méthanone;
- la 2-benzoyl-6-éthyl-7-méthylthio-imidazo[1,2-c] pyrimidir-5-one,

et leurs sels.

L'invention a également pour objet un procédé de préparation des imidazo[1,2-c] pyrimidines telles que définies par la formule (I) ci-dessus dans laquelle $R_1$ forme avec $R_2$ une fonction cétone, ainsi que de leurs sels, caractérisé en ce que l'on fait réagir au sein d'un solvant organique constitué par le tétrahydrofuranne, l'éther éthylique ou le diméthoxyéthane un produit de formule (II):

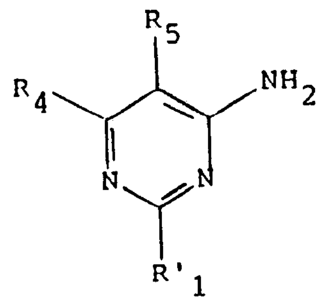

(II)

dans laquelle
$R'_1$ représente un radical alkoxy renfermant de 1 à 5 atomes de carbone,
$R_4$ et $R_5$ ont la signification déjà indiquée,
avec un produit de formule (III):

$X-CH_2-CO-CO-R$ (III)

dans laquelle
X représente un groupement nucléophile et
R a la signification déjà indiquée,
puis cyclise au reflux d'un alcanol pour obtenir un produit de formule:

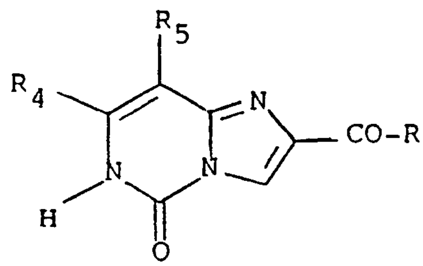

($I_A$)

dans laquelle R, $R_4$ et $R_5$ ont la signification déjà indiquée et que,
- soit on isole et, si désiré, salifie le produit de formule (I) ainsi obtenu,
- soit on fait réagir le dit produit de formule ($I_A$) obtenu avec un produit de formule (IV):

$A-R'_3$ (IV)

dans laquelle
A représente un atome d'halogène et
$R'_3$ représente un radical alkyle renfermant de 1 à 5 atomes de carbone ou un radical alcényle renfermant de 2 à 5 atomes de carbone
au sein du diméthylformamide au moyen d'un iodure ou d'alkyle ou d'alcényle en présence d'hydrure de sodium pour obtenir un produit de formule ($I_B$):

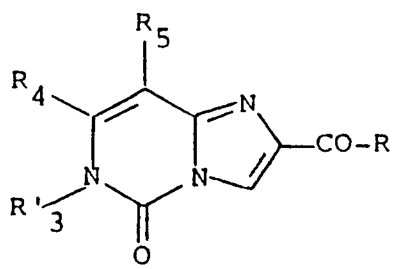

($I_B$)

dans laquelle R, $R'_3$ $R_4$ et $R_5$ ont la signification déjà indiquée et que l'on isole et, si désiré, salifie le produit de formule ($I_B$) ainsi obtenu.

Dans des conditions préférentielles de mise en oeuvre de l'invention, le procédé de préparation ci-dessus

décrit, est caractérisé en ce que:

a) le groupement nucléophile est avantageusement un groupement Br;

b) la cyclisation est effectuée au reflux d'un alcanol, par exemple à une température supérieure à 70° C, dans un solvant tel que l'éthanol;

L'invention a également pour objet un procédé de préparation des imidazo[1,2-c] pyrimidines telles que définies par la formule (I) ci-dessus dans laquelle $R_1$ représente un atome d'hydrogène ou un radical alkyle alkoxy ou alkylthio renfermant de 1 à 5 atomes de carbone ainsi que de leurs sels caractérisé en ce que l'on fait réagir au sein d'un solvant organique constitué par le tétrahydrofuranne, l'éther éthylique ou le diméthoxyéthane un produit de formule (II'):

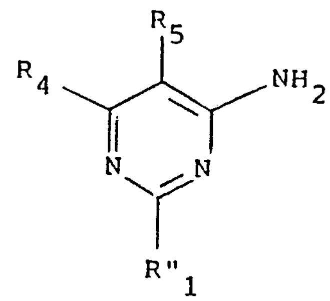

(II')

dans laquelle

$R''_1$ représente un atome d'hydrogène ou un radical alkyle, alkoxy ou alkylthio renfermant de 1 à 5 atomes de carbone et

$R_4$ et $R_5$ ont la signification déjà indiquée,

avec un produit de formule (III):

$X\text{-}CH_2\text{-}CO\text{-}CO\text{-}R$ (III)

dans laquelle

X représente un groupement nucléophile et

R a la signification déjà indiquée

puis cyclisé au reflux d'un alcanol pour obtenir un produit de formule:

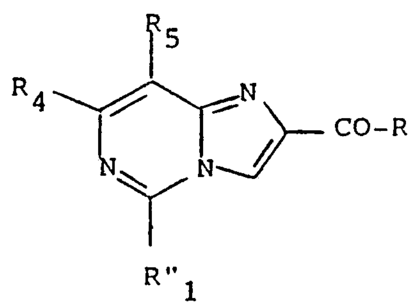

$(I_C)$

dans laquelle R, $R''_1$, $R_4$ et $R_5$ ont la signification déjà indiquée et que l'on isole et, si désiré, salifie le produit de formule ($I_C$) ainsi obtenu.

Dans des conditions préférentielles de mise en oeuvre de l'invention, le procédé de préparation ci-dessus décrit est caractérisé en ce que:

a) le groupement nucléophile est avantageusement un groupement Br.

b) la cyclisation est effectuée au reflux d'un alcanol inférieur; on opère par exemple au reflux du méthanol lorsque l'on désire préparer un produit de formule (I) dans laquelle $R_1$ représente un radical alkoxy renfermant de 1 à 5 atomes de carbone; on peut, par contre, opérer au reflux de l'éthanol lorsque l'on désire préparer un produit de formule (I) dans laquelle $R_1$ représente un atome d'hydrogène ou un radical alkyle ou alkylthio renfermant de 1 à 5 atomes de carbone.

Dans le procédé selon l'invention, l'isolement du produit obtenu avant la cyclisation n'est pas indispensable.

La réaction de cyclisation peut conduire, selon le caractère basique du produit obtenu à un sel d'acide ou à un produit non salifié. Les sels d'acides peuvent être transformés si désiré, en bases libres correspondantes, par action d'une base, par exemple un hydroxyde, un carbonate ou un bicarbonate de métal alcalin.

Comme rappelé plus haut, les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques; ils sont doués notamment de remarquables propriétés anxiolytiques.

Ces propriétés sont illustrées plus loin dans la partie expérimentale.

Ces propriétés justifient l'utilisation des imidazo[1,2-c] pyrimidines de formule (I), ainsi que de leurs sels, pharmaceutiquement acceptables, à titre de médicaments.

La présente invention a ainsi également pour objet l'application à titre de médicaments des imidazo[1,2-c] pyrimidines telles que définies par la formule générale (I) ainsi que de leurs sels d'addition avec les acides pharmaceutiquement acceptables.

Parmi les médicaments, objet de l'invention, on retient notamment les médicaments caractérisés en ce qu'ils sont constitués par les imidazo[1,2-c] pyrimidines répondant à la formule (I) dans laquelle R représente un radical phényle et notamment, parmi ceux-ci, ceux dans laquelle $R_4$ représente un radical alkoxy ou alkyithio et $R_1$, $R_2$, $R_3$ et $R_5$ ont la signification déjà indiquée, ainsi que leurs sels d'addition avec les acides pharmaceutiquement acceptables.

Parmi les médicaments de l'invention, on retient tout particulièrement ceux répondant à la formule (I) dans laquelle $R_3$ forme avec $R_2$ une seconde liaison carbone-azote ou représente un radical alkyle renfermant de 1 à 5 atomes de carbone ou un radical alcényle renfermant de 2 à 5 atomes de carbone, $R_4$ représente un radical méthoxy ou méthylthio et R représente un radical phényle, ainsi que leurs sels d'addition avec les acides pharmaceutiquement acceptables.

Parmi les médicaments préférés de l'invention, on retient tout particulièrement:
- la 2-benzoyl-6-éthyl-7-méthoxy-imidazo[1,2-c] pyrimidin-5-one;
- la 2-benzoyl-7-méthoxy-6-propyl-imidazo[1,2-c] pyrimidin-5-one;
- la (5-éthyl-7-méthoxy-imidazo[1,2-c] pyrimidin-2-yl) phényl méthanone;
- la (7-méthoxy-5-méthyl imidazo[1,2-c] pyrimidin-2-yl) phénylméthanone;
- la 6-allyl-2-benzoyl-7-méthoxyimidazo[1,2-c] pyrimidin-5-one;
- la (7-méthoxy-5-méthylthio-imidazo[1,2-c] pyrimidin-2-yl) phényl méthanone;
- la 2-benzoyl-6-éthyl-7-méthylthio-imidazo[1,2-c] pyrimidin-5-one,

et leurs sels pharmaceutiquement acceptables.

Ces médicaments trouvent, par exemple, leur emploi dans le traitement des états anxieux, des anxiétés chroniques avec agitation, irritabilité, agressivité, des anxiétés avec insomnies et tensions musculaires, des angoisses.

La dose usuelle, variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II), lorsqu'ils ne sont pas connus, peuvent être préparés par un procédé analogue à celui décrit dans Chem. Pharm. Bull, 1976, 24 (3), 507.

Les produits de formule (II'), lorsqu'ils ne sont pas connus, peuvent être préparés par des procédés analogues à ceux décrits dans J.A.C.S 73, 1951, 106, Chem. Ber. 75, 1942, 755 ou Chem. Pharm. Bull., 18, 1970, 1385.

Les produits de formule (III) dans laquelle X représente un atome d'halogène peuvent être préparés, par exemple, par halogénation d'un produit de formule (A):

$$CH_3\text{-}CO\text{-}CO\text{-}R \qquad (A)$$

selon le procédé décrit dans Helv. Chim. Acta, 29, 1946, 1247.

Les produits de formule (A) peuvent, quant à eux, être préparés par bromuration d'un produit de formule (B):

$$CH_3\text{-}CH_2\text{-}CO\text{-}R \qquad (B)$$

suivie d'un traitement par le méthylate de sodium, pour conduire au produit de formule (C):

$$\begin{array}{l} \quad\;\; OCH_3 \\ \quad\;\;\; | \\ CH_3\text{-}C\text{-}CO\text{-}R \\ \quad\;\;\; | \\ \quad\;\; OCH_3 \end{array} \qquad (C)$$

que l'on fait réagir avec un acide, par exemple l'acide chlorhydrique.

Les exemples suivants illustrent l'invention sans toutefois la limiter.

**Exemple 1:** 2-benzoyl-7-méthoxy-imidazo[1,2-c] pyrimidin-5-one.

On ajoute une solution de 21 g de 3-bromo-1-phényl propane 1,2-dione dans 50 ml d'éther éthylique à 10 g de 2,6-diméthoxy-4-pyrimidinamine en solution dans 25 ml de tétrahydrofuranne et agite pendant 16 heures à température ambiante. On ajoute 20 ml d'éther, glace et sépare par filtration 16,7 g de sel de pyridinium cristallisé. On dissout 10 g de ce sel dans 30 ml d'éthanol, chauffe 2 heures au reflux sous atmosphère inerte, refroidit, dilue avec 30 ml d'éther éthylique glace et filtre les cristaux formés. On obtient 4,4 g de produit attendu. F = 208 - 210°C.

On concentre à sec le filtrat, chromatographie le résidu sur silice en éluant à l'acétate d'éthyle et obtient un second jet de 1,4 g de produit attendu.

La 3-bromo-1-phényl propane-1,2-dione utilisée au départ de l'exemple 1 a été praparée comme suit.

**Stade A:** 1-phényl propane 1,2-dione.

On introduit en 6 heures environ 6 kg de propiophénone puis 92 g de chlorure d'aluminium dans 6 litres d'éther puis chauffe au reflux pendant 16 heures. On évapore le solvant sous pression réduite, reprend l'huile obtenue par une solution de 2,81 kg de sodium dans 45 litres de méthanol en maintenant la température inférieure à 20°C. On ajoute 12,5 litres d'acide chlorhydrique, agite 1 heure à température ambiante, filtre, concentre le filtrat jusqu'à un volume d'environ 25 litres, ajoute 10 litres de chloroforme et 10 litres d'eau, sépare la phase aqueuse et l'extrait au chloroforme. On réunit les phases chloroformiques, les sèche, les concentre sous pression réduite et distille le résidu. On recueille les fractions dont le point d'ébullition est compris entre 77°C et 85°C et obtient 5,7 kg de produit attendu.

**Stade B:** 3-bromo-1-phényl propane 1,2-dione.

On chauffe à 50°C une solution de 5,2 kg de produit obtenu au stade A dans 36 litres de chloroforme. On ajoute lentement 5,66 kg de brome en solution dans 8 litres de chloroforme et maintient 6 heures au reflux. On laisse revenir à température ambiante, lave avec une solution aqueuse saturée de bicarbonate de sodium, sèche et élimine le solvant sous pression reduite. On obtient 8,2 kg de produit attendu que l'on utilise tel quel pour le stade suivant.

**Exemple 2:** 2-benzoyl-6-éthyl-7-méthoxyimidazo[1,2-c] pyrimidin-5-one.

On ajoute 1,27 g d'hydrure de sodium à 11 g de produit obtenu à l'exemple 1 en solution dans 33 ml de diméthylformamide et agite pendant 1 heure. On ajoute en 10 minutes, 8 g d'iodure d'éthyle, agite 2 heures, verse le mélange réactionnel dans l'eau glacée, filtre et obtient 2,3 g de produit attendu. F = 159 - 160°C.

**Exemple 3:** 2-benzoyl-7-méthoxy-6-propylimidazo[1,2-c] pyrimidin-5-one.

En opérant comme à l'exemple 2 au départ de la 2-benzoyl-7-méthoxyimidazo[1,2-c] pyrimidin-5-one et de iodopropane, on obtient le produit attendu.

**Exemple 4:** (7-méthoxy-5-méthylimidazo[1,2-c] pyrimidin-2-yl) phényl méthanone.

On mélange une solution de 5,4 g de 3-bromo-1-phényl 1,2-propane dione préparée comme ci-dessus à la fin de l'exemple 1 dans 5 ml d'éther à 2,1 g de 6-méthoxy-2-méthyl 4-pyrimidinamine en solution dans le tétrahydrofuranne; On agite pendant 16 heures, glace et filtre le précipité que l'on remet en suspension dans l'éthanol et chauffe au reflux pendant 1 heure et demie. On refroidit, glace et filtre, reprend le précipité dans

un mélange de chloroforme et d'une solution aqueuse de bicarbonate de sodium. On sépare la phase organique, concentre à sec sous pression réduite et chromatographie le résidu sur silice en éluant à l'acétate d'éthyle. On obtient 2,7 g de produit attendu. F = 165 - 167° C.

**Exemples 5 à 15:**

En utilisant une méthode analogue à celle des exemples 1, 2 ou 4 (appelée respectivement A, B et C dans le tableau ci-dessous) mais en utilisant au départ les composés correspondants de formule (II) dans laquelle R, $R_1$, $R_2$, $R_3$, $R_4$ et $R_5$ ont les significations indiquées dans le tableau I ci-après ou les composés correspondants de formule (I) dans laquelle $R_1$ et $R_2$ représentent une fonction cétone, $R_3$ un atome d'hydrogène et R, $R_4$ et $R_5$ ont les significations indiquées dans le tableau I, on a préparé les composés des exemples 5 à 9 et 12 à 15.

Le composé de l'exemple 10 a été préparé par une méthode (appelée A* dans le tableau ci-dessous) analogue à celle de l'exemple 1, mais en utilisant le méthanol à la place de l'éthanol lors de la cyclisation.

Le composé de l'exemple 11 a été obtenu comme sous-produit de l'exemple 2 mais peut être également préparé avec de meilleurs rendements par la méthode A*.

Les analyses spectométriques, les rendements, les points de fusion et les résultats de la microanalyse sont donnés dans le tableau I.

**Exemple 5:** (5-éthyl-7-méthoxyimidazo[1,2-c] pyrimidin-2-yl) phényl méthanone.

**Exemple 6:** 2-benzoyl-7-éthoxyimidazo[1,2-c] pyrimidin-5-one.

**Exemple 7:** 2-benzoyl-7-méthoxy-6-méthylimidazo[1,2-c] pyrimidin-5-one.

**Exemple 8:** (7-méthoxy-5,8-diméthylimidazo[1,2-c] pyrimidin-2-yl) phényl méthanone.

**Exemple 9:** 6-allyl-2-benzoyl-7-méthoxyimidazo[1,2-c] pyrimidin-5-one.

**Exemple 10:** (5,7-diméthoxyimidazo[1,2-c] pyrimidin-2-yl) phényl méthanone.

**Exemple 11:** (5-éthoxy-7-méthoxyimidazo[1,2-c] pyrimidin-2-yl) phényl méthanone.

**Exemple 12:** [7-méthoxy-5-(méthylthio) imidazo[1,2-c] pyrimidin-2-yl] phényl méthanone.

**Exemple 13:** [5,7-bis (méthylthio) imidazo[1,2-c] pyrimidin-2-yl] phényl méthanone.

**Exemple 14:** 2-benzoyl-6-éthyl-7-(méthylthio) imidazo[1,2-c] pyrimidin-5-one.

**Exemple 15:** 6-allyl-2-benzoyl-7-(méthylthio) imidazo[1,2-c] pyrimidin-5-one.

**Tableau I**

| Ex. n° | R | $R_1$ | $R_2$ | $R_3$ | $R_4$ | $R_5$ | Méthode | Rendement % |
|---|---|---|---|---|---|---|---|---|
| 1 | Ph | =O | | H | $CH_3O$ | H | A | 64 |
| 2 | Ph | =O | | $C_2H_5$ | $CH_3O$ | H | B | 19 |
| 3 | Ph | =O | | $C_3H_7$ | $CH_3O$ | H | B | 18 |
| 4 | Ph | $CH_3$ | = = | | $CH_3O$ | H | C | 67 |
| 5 | Ph | $C_2H_5$ | = = | | $CH_3O$ | H | C | 45 |
| 6 | Ph | =O | | H | $C_2H_5O$ | H | A | 20 |
| 7 | Ph | =O | | $CH_3$ | $CH_3O$ | H | B | 40 |
| 8 | Ph | $CH_3$ | = = | | $CH_3O$ | $CH_3$ | C | 44 |
| 9 | Ph | =O | | $CH_2=CHCH_2$ | $CH_3O$ | H | B | 26 |
| 10 | Ph | $CH_3O$ | = = | | $CH_3O$ | H | A* | 71 |
| 11 | Ph | $C_2H_5O$ | = = | | $CH_3O$ | H | B | 0.3 |
| 12 | Ph | $CH_3S$ | = = | | $CH_3O$ | H | C | 29 |
| 13 | Ph | $CH_3S$ | = = | | $CH_3S$ | H | C | 25 |
| 14 | Ph | =O | | $C_2H_5$ | $CH_3S$ | H | B | 47 |
| 15 | Ph | =O | | $CH_2=CH-CH_2$- | $CH_3S$ | H | B | 52 |

**Tableau I** (Suite)

| Ex. N° | IR $cm^{-1}$ (KBr) | F°C | Formule | Poids Mol. |
|---|---|---|---|---|
| 1 | 1640,1670,1650,1610 | 208 - 10°C | $C_{14}H_{11}N_3O_3$ | 269.3 |
| 2 | 1700,1640,1600,1580 | 159 - 60°C | $C_{16}H_{15}N_3O_3$ | 297.3 |
| 3 | 1710,1630,1600,1580 | 106 - 7°C | $C_{17}H_{17}N_3O_3$ | 311.3 |
| 4 | 1645,1635,1595,1575 | 165 - 7°C | $C_{15}H_{13}N_3O_2$ | 267.3 |
| 5 | 1640,1600,1560,1500 | 152 - 3°C | $C_{16}H_{15}N_3O_2$ | 281.3 |
| 6 | 1730,1670,1650,1600 | 218 - 20°C | $C_{15}H_{13}N_3O_3$ | 283.3 |
| 7 | 1710,1625,1600,1570 | 187 - 9°C | $C_{15}H_{13}N_3O_3$ | 283.3 |
| 8 | 1640,1630,1600,1580 | 185 - 7°C | $C_{16}H_{15}N_3O_2$ | 281.3 |
| 9 | 1710,1660,1630,1590 | 139 - 41°C | $C_{17}H_{15}N_3O_3$ | 309.3 |
| 10 | 1650,1630,1600,1570 | 141 - 3°C | $C_{15}H_{13}N_3O_3$ | 283.3 |
| 11 | 1660,1640,1600,1590 | 116 - 8°C | $C_{16}H_{15}N_3O_3$ | 297.3 |
| 12 | 1640,1620,1515,1460 | 149 - 50°C | $C_{15}H_{13}N_3O_2S$ | 299.35 |
| 13 | 1640,1600,1580,1520 | 154 - 5°C | $C_{15}H_{13}N_3OS_2$ | 315.4 |
| 14 | 1680,1635,1600,1530 | 152 - 4°C | $C_{16}H_{15}N_3O_2S$ | 313.4 |
| 15 | 1690,1635,1595 | 161°C | $C_{17}H_{15}N_3O_2S$ | 325.4 |

**Tableau I** (suite)

| Ex. | Calculé/Trouvé | | | |
|---|---|---|---|---|
| n° | C | H | N | S |
| 1 | 62.45/62.19 | 4.12/4.15 | 15.16/15.38 | |
| 2 | 64.64/64.66 | 5.09/5.09 | 14.13/14.15 | |
| 3 | 65.58/65.59 | 5.51/5.77 | 13.50/13.38 | |
| 4 | 67.41/67.2 | 4.90/4.98 | 15.72/15.65 | |
| 5 | 68.31/68.19 | 5.37/5.44 | 14.94/14.91 | |
| 6 | 63.60/63.58 | 4.62/4.58 | 14.83/14.56 | |
| 7 | 63.60/63.47 | 4.62/4.70 | 14.68/14.87 | |
| 8 | 68.31/68.38 | 5.37/5.16 | 14.94/14.96 | |
| 9 | 66.01/65.82 | 4.89/4.97 | 13.58/13.56 | |
| 10 | 63.60/63.73 | 4.62/4.63 | 14.83/14.90 | |
| 11 | 64.64/64.55 | 5.09/5.08 | 14.13/14.19 | |
| 12 | 60.19/60.18 | 4.38/4.43 | 14.04/14.06 | 10.71/10.63 |
| 13 | 57.12/57.28 | 4.15/4.20 | 13.32/13.32 | 20.33/20.06 |
| 14 | 61.32/61.70 | 4.82/4.86 | 13.41/13.38 | 10.23/10.29 |
| 15 | 62.75/62.77 | 4.65/4.73 | 12.93/12.86 | 9.85/9.77 |

**Exemple 16:**

On a préparé des comprimés correspondant à la formulation suivante:

- Composé de l'exemple 2 20 mg
- Excipient q.s. pour un comprimé terminé à 150 mg

(Détail de l'excipient: lactose, amidon, talc, stéarate de magnésium).

**Exemple 17:**

On a préparé des comprimés correspondant à la formulation suivante:

- Composé de l'exemple 5 20 mg
- Excipient q.s. pour un comprimé terminé à 150 mg

(Détail de l'excipient: lactose, amidon, talc, stéarate de magnésium).

**Activities biochimiques et pharmacologiques**

1) Activité biochimique

L'affinité des composés pour les récepteurs des benzodiazépines été évaluée en utilisant un radioligand /3H/ flunitrazépam et la méthode de Squires et Braestrup (Nature, 266, 1977, 732) modifiée.

Les valeurs indiquées dans le tableau II ci-après sont les concentrations nanomolaires du produit testé qui inhibent dans une proportion de 50 % la liaison spécifique de 0,6 nanomoles (3H) flunitrazépam dans des préparations des membranes de cerveaux antérieurs de rats ($CI_{50}$ en nanomoles).

2) Activité pharmacologique

L'étude de l'activité anxyolitique des composés a été effectuée par une méthode selon Geller et Seifter (Psychopharmacologia, 1960, I, 482) modifiée.

Les valeurs données dans le tableau II sont les doses efficaces minimum auxquelles il est observé un accroissement des chocs par rapport aux témoins (DEM mg/kg po).

**Tableau II**

| Exemple n° | Liaison aux récepteurs $CI_{50}$nM | Méthode de Geller (DEM mg/kg po) |
|---|---|---|
| 2 | 2000 | 2 |
| 3 | 1000 | 10 |
| 4 | 520 | 50 |
| 5 | 215 | 10 |
| 9 | 500 | 5 |
| 10 | 1000 | 10 |
| 11 | 890 | - |
| 12 | 126 | 5 |
| 13 | 18 | 50 |
| 14 | 140 | 5 |
| 15 | 120 | 50 |

**Revendications**

1. Imidazo[1,2-c] pyrimidines répondant à la formule (I):

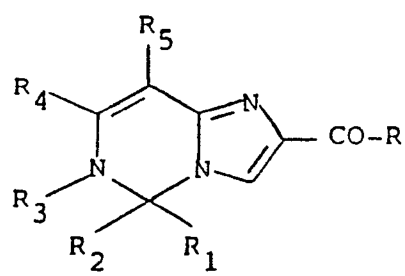

(I)

dans laquelle

R représente un radical aryle renfermant de 6 à 12 atomes de carbone,

$R_1$ représente un atome d'hydrogène, un radical alkyle, alkoxy ou alkylthio renfermant de 1 à 5 atomes de carbone ou forme avec $R_2$ une fonction cétone,

$R_2$ forme avec $R_1$ une fonction cétone ou $R_2$ forme avec $R_3$ une seconde liaison carbone-azote,

$R_3$ forme avec $R_2$ une seconde liaison carbone-azote ou $R_3$ représente un atome d'hydrogène, un radical alkyle renfermant de 1 à 5 atomes de carbone ou un radical alcényle renfermant de 2 à 5 atomes de carbone,

$R_4$ représente un radical alkoxy renfermant de 1 à 5 atomes de carbone ou un radical alkylthio renfermant de 1 à 5 atomes de carbone,

$R_5$ représente un atome d'hydrogène ou un radical alcoyle renfermant de 1 à 5 atomes de carbone,

ainsi que leurs sels d'addition avec les acides minéraux ou organiques.

2. Imidazo[1,2-c] pyrimidines telles que définies à la revendication 1, et leurs sels, caractérisées en ce que dans ladite formule (I), R représente un radical phényle, $R_1$, $R_2$, $R_3$, $R_4$ et $R_5$ ont la signification déjà indiquée.

3. Imidazo[1,2-c] pyrimidines telles que définies à la revendication 1 ou 2, et leurs sels, caractérisées en ce que dans ladite formule (I), $R_4$ représente un radical méthoxy ou méthylthio et R, $R_1$, $R_2$, $R_3$ et $R_5$ ont la signification déjà indiquée.

4. Imidazo[1,2-c] pyrimidines telles que définies à la revendication 1, 2 ou 3 et leurs sels, caractérisées en ce que dans ladite formule (I), $R_3$ forme avec $R_2$ une seconde liaison carbone-azote ou représente un radical alkyle renfermant de 1 à 5 atomes de carbone ou un radical alcényle renfermant de 2 à 5 atomes de carbone, $R_4$ représente un radical méthoxy ou méthylthio et R représente un radical phényle.

5. L'un quelconque des produits de formule (I) telle que définie à la revendication 1, dont lés noms suivent:
- la 2-benzoyl-6-éthyl-7-méthoxy-imidazo[1,2-c] pyrimidin-5-one;
- la 2-benzoyl-7-méthoxy-6-propyl-imidazo[1,2-c] pyrimidin-5-one;
- la (5-éthyl-7-méthoxy-imidazo[1,2-c] pyrimidin-2-yl) phényl méthanone;
- la (7-méthoxy-5-méthyl imidazo[1,2-c] pyrimidin-2-yl) phényl méthanone;
- la 6-allyl-2-benzoyl-7-méthoxyimidazo[1,2-c] pyrimidin-5-one;
- la (7-méthoxy-5-méthylthio-imidazo[1,2-c] pyrimidin-2-yl) phényl méthanone;
- la 2-benzoyl-6-éthyl-7-méthylthio-imidazo[1,2-c] pyrimidin-5-one, et leurs sels.

6. Procédé de préparation des imidazo[1,2-c] pyrimidines telles que définies par la formule (I) de la revendication 1, dans laquelle $R_1$ forme avec $R_2$ une fonction cétone, ainsi que de leurs sels, caractérisé en ce que l'on fait réagir au sein d'un solvant organique constitué par le tétrahydrofuranne, l'éther éthylique ou le diméthoxyéthane un produit de formule (II):

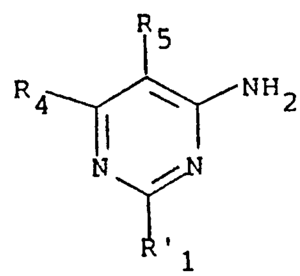

(II)

dans laquelle
$R'_1$ représente un radical alkoxy renfermant de 1 à 5 atomes de carbone,
$R_4$ et $R_5$ ont la signification déjà indiquée,
avec un produit de formule (III):

$X\text{-}CH_2\text{-}CO\text{-}CO\text{-}R$ (III)

dans laquelle
X représente un groupement nucléophile et
R a la signification déjà indiquée,
puis cyclisé au reflux d'un alcanol pour obtenir un produit de formule:

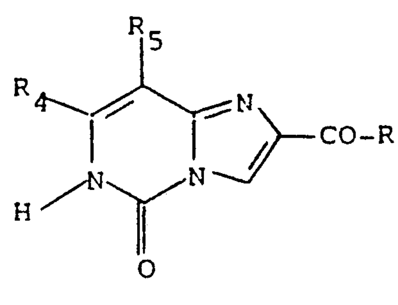

$(I_A)$

dans laquelle R, $R_4$ et $R_5$ ont la signification déjà indiquée et que,
- soit on isole et, si désiré, salifie le produit de formule (I) ainsi obtenu,
- soit on fait réagir le dit produit de formule $(I_A)$ obtenu avec un produit de formule (IV):

$A\text{-}R'_3$ (IV)

dans laquelle
A représente un atome d'halogène et
$R'_3$ représente un radical alkyle renfermant de 1 à 5 atomes de carbone ou un radical alcényle renfermant de 2 à 5 atomes de carbone
au sein du diméthylformamide au moyen d'un iodure d'alkyle ou d'alcényle, en présence d'hydrure de sodium pour obtenir un produit de formule $(I_B)$:

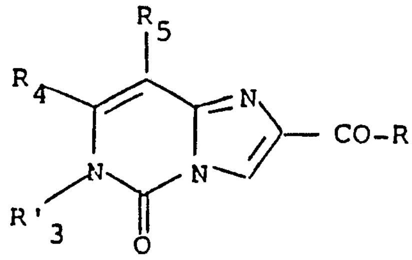

$(I_B)$

dans laquelle R, $R'_3$, $R_4$ et $R_5$ ont la signification déjà indiquée et que l'on isole et, si désiré, salifie le produit de formule $(I_B)$ ainsi obtenu.

7. Procédé de préparation selon la revendication 6, caractérisé en ce que:

a) le groupement nucléophile est un groupement Br;

b) la cyclisation est effectuée au reflux d'un alcanol, à une température supérieure à 70° c.

8. Procédé de préparation des imidazo[1,2-c] pyrimidines telles que définies par la formule (I) de la revendication 1, dans laquelle $R_1$ représente un atome d'hydrogène ou un radical alkyle, alkoxy ou alkylthio renfermant de 1 à 5 atomes de carbone, ainsi que de leurs sels, caractérisé en ce que l'on fait réagir au sein d'un solvant organique constitué par le tétrahydrofuranne, l'éther éthylique ou le diméthoxyéthane, un produit de formule (II'):

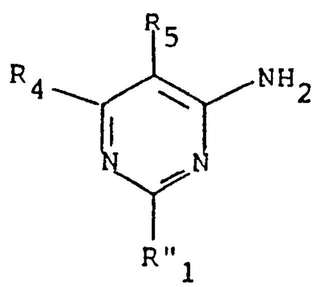

(II')

dans laquelle

$R''_1$ représente un atome d'hydrogène ou un radical alkyle, alkoxy ou alkylthio renfemant de 1 à 5 atomes dé carbone et

$R_4$ et $R_5$ ont la signification déjà indiquée,

avec un produit de formule (III):

$X\text{-}CH_2\text{-}CO\text{-}CO\text{-}R$ (III)

dans laquelle

X représente un groupement nucléophile et

R a la signification déjà indiquée,

puis cyclisé au reflux d'un alcanol pour obtenir un produit de formule:

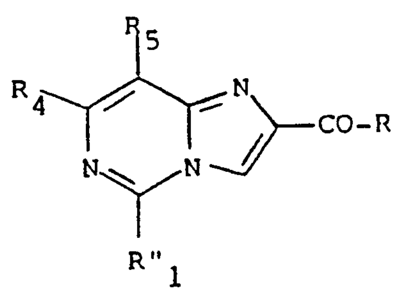

$(I_C)$

dans laquelle R, $R''_1$, $R_4$ et $R_5$ ont la signification déjà indiquée et que l'on isole et, si désiré, salifie le produit de formule $(I_C)$ ainsi obtenu.

9. Procédé de préparation selon la revendication 8, caractérisé en ce que:

a) le groupement nucléophile est un groupement Br.

b) la cyclisation est effectuée au reflux d'un alcanol inférieur.

10. Médicaments, caractérisés en ce qu'ils sont constitués par les imidazo[1,2-c] pyrimidines telles que définies par la formule (I) de la revendication 1, ainsi que par leurs sels pharmaceutiquement acceptables.

11. Médicaments, caractérisés en ce qu'ils sont constitués par les imidazo[1,2-c] pyrimidines, telles que définies à l'une quelconque des revendications 2, 3, 4 ou 5, ainsi que par leurs sels pharmaceutiquement acceptables.

12. Compositions pharmaceutiques, caractérisées en ce qu'elles renferment à titre de principe actif, l'un au moins des médicaments tels que définis à l'une quelconque des revendications 10 ou 11.

**Patentansprüche**

1. Imidazo[1,2-c]pyrimidine der Formel (I)

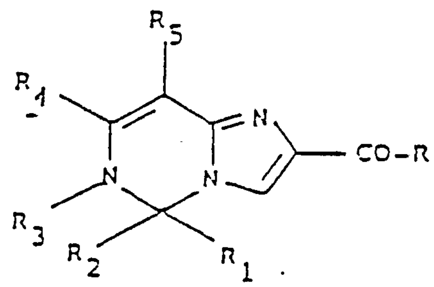

(I)

worin

R für einen Arylrest mit 6 bis 12 Kohlenstoffatomen steht,

$R_1$ ein Wasserstoffatom, einen Alkyl-, Alkoxy- oder Alkylthiorest mit 1 bis 5 Kohlenstoffatomen darstellt oder mit $R_2$ eine Ketofunktion bildet,

$R_2$ mit $R_1$ eine Ketofunktion bildet oder $R_2$ mit $R_3$ eine zweite Kohlenstoff-Stickstoff-Bindung bildet,

$R_3$ mit $R_2$ eine zweite Kohlenstoff-Stickstoff-Bindung bildet oder $R_3$ für ein Wasserstoffatom, einen Alkylrest mit 1 bis 5 Kohlenstoffatomen oder einen Alkenylrest mit 2 bis 5 Kohlenstoffatomen steht,

$R_4$ einen Alkoxyrest mit 1 bis 5 Kohlenstoffatomen oder einen Alkylthiorest mit 1 bis 5 Kohlenstoffatomen wiedergibt,

$R_5$ ein Wasserstoffatom oder einen Alkylrest mit 1 bis 5 Kohlenstoffatomen bedeutet,

sowie deren Additionssalze mit Mineral- oder organischen Säuren.

2. Imidazo[1,2-c]pyrimidine gemäß Anspruch 1 und deren Salze, dadurch gekennzeichnet, daß in der Formel (I) R für einen Phenylrest steht, $R_1$, $R_2$, $R_3$, $R_4$ und $R_5$ die angegebene Bedeutung besitzen.

3. Imidazo[1,2-c]pyrimidine gemäß Anspruch 1 oder 2 und deren Salze, dadurch gekennzeichnet, daß in der Formel (I) $R_4$ eine Methoxy- oder Methylthiogruppe bedeutet und R, $R_1$, $R_2$, $R_3$ und $R_5$ die angegebene Bedeutung besitzen.

4. Imidazo[1,2-c]pyrimidine gemäß Anspruch 1, 2 oder 3 und deren Salze, dadurch gekennzeichnet, daß in der Formel (I) $R_3$ mit $R_2$ eine zweite Kohlenstoff-Stickstoff-Bindung bildet oder einen Alkylrest mit 1 bis 5 Kohlenstoffatomen oder einen Alkenylrest mit 2 bis 5 Kohlenstoffatomen wiedergibt, $R_4$ für eine Methoxy- oder Methylthiogruppe steht und R einen Phenylrest bedeutet.

5. Eines der Produkte der Formel (I) gemäß Anspruch 1 mit den folgenden Bezeichnungen:

2-Benzoyl-6-ethyl-7-methoxy-imidazo[1,2-c]pyrimidin-5-on;

2-Benzoyl-7-methoxy-6-propyl-imidazo[1,2-c]pyrimidin-5-on;

(5-Ethyl-7-methoxy-imidazo[1,2-c]pyrimidin-2-yl)-phenylmethanon;

(7-Methoxy-5-methyl-imidazo[1,2-c]pyrimidin-2-yl)-phenylmethanon;

6-Allyl-2-benzoyl-7-methoxy-imidazo[1,2-c]pyrimidin-5-on;

(7-Methoxy-5-methylthio-imidazo[1,2-c]pyrimidin-2-yl)-phenylmethanon;

2-Benzoyl-6-ethyl-7-methylthio-imidazo[1,2-c]pyrimidin-5-on

sowie deren Salze.

6. Verfahren zur Herstellung von Imidazo[1,2-c]pyrimidinen der Formel (I) gemäß Anspruch 1, worin $R_1$ mit $R_2$ eine Ketofunktion bildet, sowie von deren Salzen, dadurch gekennzeichnet, daß man in dem Medium eines organischen Lösungsmittels, bestehend aus Tetrahydrofuran, Ethylether oder Dimethoxyethan, ein Produkt der Formel (II)

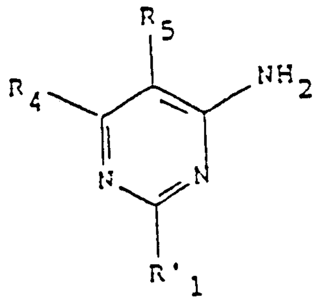

(II)

worin
$R'_1$ für eine Alkoxygruppe mit 1 bis 5 Kohlenstoffatomen steht,
$R_4$ und $R_5$ die angegebene Bedeutung besitzen,
mit einem Produkt der Formel (III)

$X\text{-}CH_2\text{-}CO\text{-}CO\text{-}R$ (III)

worin
X für eine nucleophile Gruppe steht und
R die angegebene Bedeutung besitzt,
umsetzt, hiernach unter Rückfluß eines Alkanols cyclisiert, um zu einem Produkt der Formel

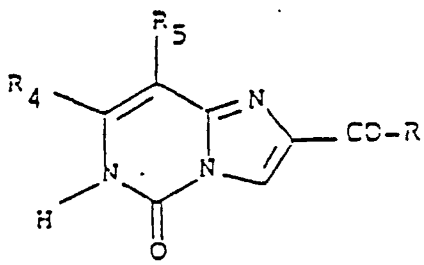

($I_A$)

zu gelangen, worin R, $R_4$ und $R_5$ die angegebene Bedeutung besitzen, und daß man
entweder das so erhaltene Produkt der Formel (I) isoliert und gewünschtenfalls in ein Salz überführt,
oder das erhaltene Produkt der Formel ($I_A$) mit einem Produkt der Formel (IV)

$A - R'_3$ (IV)

worin
A für ein Halogenatom steht und
$R'_3$ einen Alkylrest mit 1 bis 5 Kohlenstoffatomen oder einen Alkenylrest mit 2 bis 5 Kohlenstoffatomen wiedergibt,
in einem Dimethylformamid-Medium mit Hilfe eines Alkyl- oder Alkenyljodids in Anwesenheit von Natriumhydrid umsetzt, um zu einem Produkt der Formel ($I_B$)

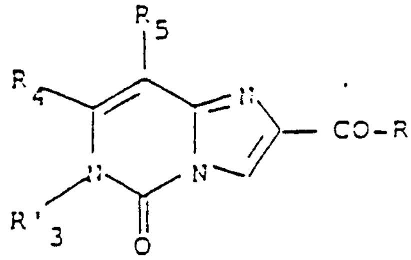

($I_B$)

zu gelangen worin R, $R'_3$, $R_4$ und $R_5$ die angegebene Bedeutung besitzen und daß man gewünschtenfalls das so erhaltene Produkt der Formel ($I_B$) in ein Salz überführt.

7. Verfahren gemäß Anspruch 6, dadurch gekennzeichnet, daß
(a) die nucleophile Gruppe eine Gruppe Br ist;
(b) die Cyclisierung unter Rückfluß eines Alkanols bei einer Temperatur oberhalb 70° C durchgeführt wird.

8. Verfahren zur Herstellung von Imidazo[1,2-c]pyrimidinen der Formel (I) gemäß Anspruch 1, worin $R_1$ für ein

Wasserstoffatom oder einen Alkyl-, Alkoxy- oder Alkylthiorest mit 1 bis 5 Kohlenstoffatomen steht, sowie von deren Salzen, dadurch gekennzeichnet, daß man in dem Medium eines organischen Lösungsmittels, bestehend aus Tetrahydrofuran, Ethylether oder Dimethoxyethan, ein Produkt der Formel (II')

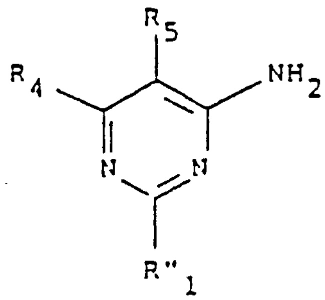

(II')

worin

R''$_1$ für ein Wasserstoffatom oder einen Alkyl-, Alkoxy- oder Alkylthiorest mit 1 bis 5 Kohlenstoffatomen steht und

$R_4$ und $R_5$ die angegebene Bedeutung besitzen,

mit einem Produkt der Formel (III)

$X\text{-}CH_2\text{-}CO\text{-}CO\text{-}R$ (III)

worin

X eine nucleophile Gruppe bedeutet und

R die angegebene Bedeutung besitzt,

umsetzt, hiernach unter Rückfluß eines Alkanols cyclisiert, um zu einem Produkt der Formel

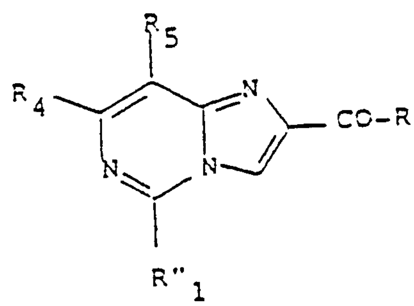

($I_C$)

zu gelangen, worin R, R''$_1$, $R_4$ und $R_5$ die angegebene Bedeutung besitzen, und daß man das so erhaltene Produkt der Formel ($I_C$) isoliert und gewünschtenfalls in ein Salz überführt.

9. Verfahren gemäß Anspruch 8, dadurch gekennzeichnet, daß

(a) die nucleophile Gruppe eine Gruppe Br ist;

(b) die Cyclisierung unter Rückfluß eines niedrigen Alkanols erfolgt.

10. Arzneimittel, dadurch gekennzeichnet, daß sie aus den Imidazo[1,2-c]pyrimidinen der Formel (I) gemäß Anspruch 1 oder aus deren pharmazeutisch annehmbaren Salzen bestehen.

11. Arzneimittel, dadurch gekennzeichnet, daß sie aus den Imidazo[1,2-c]pyrimidinen gemäß einem der Ansprüche 2, 3, 4 oder 5 oder aus deren pharmazeutisch verträglichen Salzen bestehen.

12. Pharmazeutische Zusammensetzungen, dadurch gekennzeichnet, daß sie als Wirkstoff zumindest eines der Arzneimittel gemäß einem der Ansprüche 10 oder 11 enthalten.

## Claims

1. Imidiazo [1,2-c] pyrimidines corresponding to formula (I):

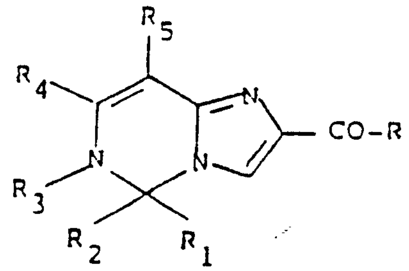

(I)

in which

R represents an aryl radical containing from 6 to 12 carbon atoms,

$R_1$ represents a hydrogen atom, an alkyl, alkoxy or alkylthio radical containing from 1 to 5 carbon atoms or forms a ketone function with $R_2$,

$R_2$ forms a ketone function with $R_1$ or $R_2$ forms a second carbon-nitrogen bond with $R_3$,

$R_3$ forms a second carbon-nitrogen bond with $R_2$ or $R_3$ represents a hydrogen atom, an alkyl radical containing from 1 to 5 carbon atoms or an alkenyl radical containing from 2 to 5 carbon atoms,

$R_4$ represents an alkoxy radical containing from 1 to 5 carbon atoms or an alkylthio radical containing from 1 to 5 carbon atoms,

$R_5$ represents a hydrogen atom or an alkyl containing from 1 to 5 carbon atoms,

as well as their addition salts with mineral or organic acids.

2. Imidazo [1,2-c] pyrimidines as defined in claim 1, and their salts, characterized in that in the said formula (I), R represents a phenyl radical, $R_1$, $R_2$, $R_3$, $R_4$ and $R_5$ have the meanings already given.

3. Imidazo [1,2-c] pyrimidines as defined in claims 1 or 2, and their salts, characterized in that in the said formula (I), $R_4$ represents a methoxy or methylthio radical and R, $R_1$, $R_2$, $R_3$ and $R_5$ have the meanings already given.

4. Imidazo [1,2-c] pyrimidines as defined in claims 1, 2 or 3, and their salts, characterized in that in the said formula (I), $R_3$ forms a second carbon-nitrogen bond with $R_2$ or represents an alkyl radical containing from 1 to 5 carbon atoms or an alkenyl radical containing from 2 to 5 carbon atoms, $R_4$ represents a methoxy or methylthio radical and R represents a phenyl radical.

5. Any one of the products from formula (I) as defined in claim 1, which have the following names:

- 2-benzoyl-6-ethyl-7-methoxy-imidazo [1,2-c] pyrimidin-5-one;
- 2-benzoyl-7-methoxy-6-propyl-imidazo [1,2-c] pyrimidin-5-one;
- (5-ethyl-7-methoxy-imidazo [1,2-c] pyrimidin-2-yl)phenyl methanone;
- (7-methoxy-5-methyl imidazo [1,2-c] pyrimidin-2-yl)phenyl methanone;
- 6-allyl-2-benzoyl-7-methoxyimidazo [1,2-c] pyrimidin-5-one;
- (7-methoxy-5-methylthio-imidazo [1,2-c] pyrimidin-2-yl)phenyl methanone;
- 2-benzoyl-6-ethyl-7-methythio-imidazo [1,2-c] pyrimidin-5-one, and their salts.

6. Preparation process for imidazo [1,2-c] pyrimidines as defined by formula (I) of claim 1, in which $R_1$ forms a ketone function with $R_2$, as well as their salts, characterized in that a product of formula (II):

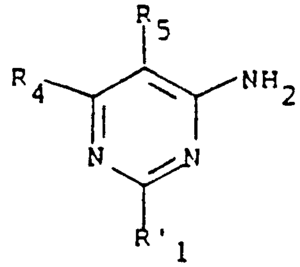

in which

$R'_1$ represents an alkoxy radical containing from 1 to 3 carbon atoms, and

$R_4$ and $R_5$ have the meanings already given,

is reacted in an organic solvent constituted by tetrahydrofuran, ethyl ether or dimethoxyethane, with a product of formula (III):

$X\text{-}CH_2\text{-}CO\text{-}CO\text{-}R$ (III)

in which

X represents a nucleophilic group and

R has the meaning already given,

then is cyclised at reflux of an alkanol to obtain a product of formula:

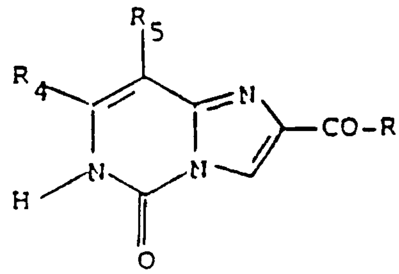

($I_A$)

in which R, $R_4$ and R5 have the meanings already given and,
- either the product of formula (I) thus obtained is isolated and, if desired, is salified,
- or the said product of formula ($I_A$) thus obtained is reacted with a product of formula (IV):

A-$R'_3$ (IV)

in which
A represents a halogen atom and
$R'_3$ represents an alkyl radical containing from 1 to 5 carbon atoms or an alkenyl radical containing from 2 to 5 carbon atoms,
in dimethyl formamide by means of an alkyl or alkenyl iodide, in the presence of sodium hydride to obtain a product of formula ($I_B$):

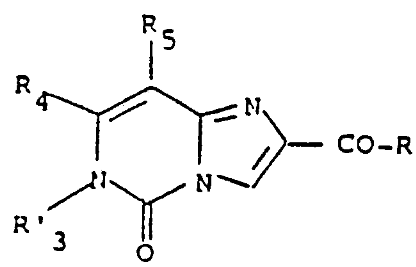

($I_B$)

in which R, $R'_3$, $R_4$ and $R_5$ have the meaning already given and the product of formula ($I_B$) thus obtained is isolated and, if desired, salified.

7. Preparation process according to claim 6, characterized in that:
a) the nucleophilic group is a Br group;
b) the cyclisation is carried out at reflux of an alkanol, at a temperature higher than 70° C.

8. Preparation process for imidazo [1,2-c] pyrimidines as defined in formula (I) of claim 1, in which $R_1$ represents a hydrogen atom or an alkyl, alkoxy or alkylthio radical containing from 1 to 5 carbon atoms, as well as their salts, characterized in that a product of formula (II'):

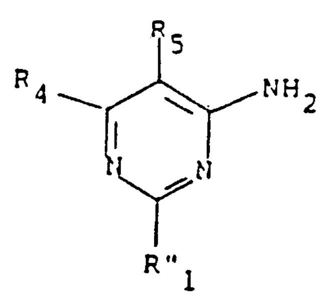

(II')

in which
$R''_1$ represents a hydrogen atom or an alkyl, alkoxy or alkylthio radical containing from 1 to 5 carbon atoms and
$R_4$ and $R_5$ have the meanings already given,
is reacted in an organic solvent constituted by tetrahydrofuran, ethyl ether or dimethoxyethane, with a product of formula (III):

X-$CH_2$-CO-CO-R (III)

in which
X represents a nucleophilic group and
R has the meaning already given,
then cyclised at reflux of an alkanol to obtain a product of formula:

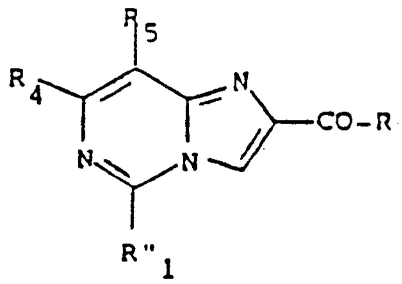

($I_C$)

in which R, R'', $R_4$ and $R_5$ have the meaning already given and the product of formula ($I_C$) thus obtained is isolated and, if desired, is salified.

9. Preparation process according to claim 8, characterized in that:
a) the nucleophilic group is a Br group,
b) the cyclisation is carried out at reflux of a lower alkohol.

10. Medicaments, characterized in that they are constituted by imidazo [1,2-c] pyrimidines, as defined by formula (I) of claim 1, as well as their pharmaceutically acceptable salts.

11. Medicaments, characterized in that they are constituted by imidiazo [1,2-c] pyrimidines, as defined in any of the claims 2, 3, 4 or 5, as well as their pharmaceutically acceptable salts.

12. Pharmaceutical compositions, characterized in that they contain, as active principle, at least one of the medicaments as defined in either of claims 10 or 11.